(19) Europäisches Patentamt
European Patent Office
Office européen des brevets

(11) Veröffentlichungsnummer: **0 213 570 B1**

(12) **EUROPÄISCHE PATENTSCHRIFT**

(45) Veröffentlichungstag der Patentschrift: **08.04.92**

(51) Int. Cl.[5]: **C07D 487/22**, C07D 519/00, C08L 61/20, C08K 5/34, //(C07D487/22,251:00,251:00, 235:00,235:00),(C07D519/00, 487:00,487:00)

(21) Anmeldenummer: **86111624.2**

(22) Anmeldetag: **22.08.86**

Die Akte enthält technische Angaben, die nach dem Eingang der Anmeldung eingereicht wurden und die nicht in dieser Patentschrift enthalten sind.

(54) **Glykolurilderivate und ihre Verwendung als Stabilisatoren für Polymere.**

(30) Priorität: **28.08.85 DE 3530666**

(43) Veröffentlichungstag der Anmeldung:
**11.03.87 Patentblatt 87/11**

(45) Bekanntmachung des Hinweises auf die Patenterteilung:
**08.04.92 Patentblatt 92/15**

(84) Benannte Vertragsstaaten:
**CH DE FR GB IT LI**

(56) Entgegenhaltungen:
**WO-A-81/01706**
**DE-A- 2 636 143**
**FR-A- 2 291 203**

**THE JOURNAL OF ORGANIC CHEMISTRY, Band 50, Nr. 1, 11. Januar 1985, Seiten 60-62, American Chemical Society; W.L. MOCK et al.: "A novel hexacyclic ring system from glycoluril"**

(73) Patentinhaber: **BASF Aktiengesellschaft**
**Carl-Bosch-Strasse 38**
**W-6700 Ludwigshafen(DE)**

(72) Erfinder: **Helwig, Reinhard, Dr.**
**Bruesseler Ring 4**
**W-6700 Ludwigshafen(DE)**
Erfinder: **Aumueller, Alexander, Dr.**
**Brechlochstrasse 9**
**W-6700 Ludwigshafen(DE)**
Erfinder: **Neumann, Peter, Dr.**
**Franz-Schubert-Strasse 1**
**W-6908 Wiesloch(DE)**
Erfinder: **Trauth, Hubert**
**Milanstrasse 6**
**W-6724 Dudenhofen(DE)**



Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Es ist bekannt, daß 2,2,6,6-Tetraalkyl-piperidinderivate Lichtschutzmittel für organische Polymere sind, beispielsweise aus der WO 81/01706, in der Tetraalkyl-4-piperidyldiamine mit einer Dialkylenamin-Brücke zwischen den beiden Piperidylaminresten, wobei die Brücken-Aminfunktion auch ein Piperazinring sein kann, beschrieben werden. Unbefriedigend ist oft die Verträglichkeit mit Polyolefinen, die Dauer der Schutzwirkung, die Neigung zur Flüchtigkeit und die Eigenfarbe der Substanzen.

Die Erfindung betrifft neue Verbindungen der allgemeinen Formel (I)

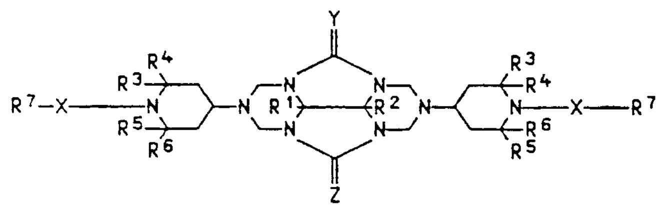

(I) ,

in der

$R^1$ und $R^2$ unabhängig voneinander Wasserstoff, $C_1$- bis $C_6$-Alkyl, $C_7$- bis $C_{12}$-Aralkyl, Phenyl, Tolyl, Carbomethoxy, Carboethoxy, Carbopropoxy oder Carbobutoxy,

$R^1$ und $R^2$ zusammen eine Tetra- oder Pentamethylengruppe,

$R^3$ bis $R^6$ $C_1$- bis $C_4$-Alkyl, wobei zwei benachbarte Rest dabei auch eine Tetra- oder Pentamethylengruppe bilden können, die Reste

X unabhängig voneinander eine direkte Bindung oder eine zweiwertige Gruppierung der Formel

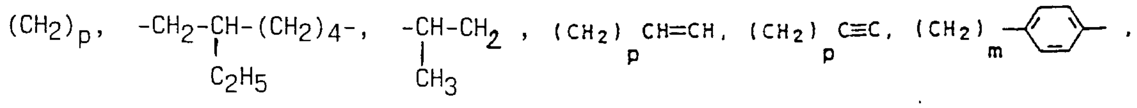

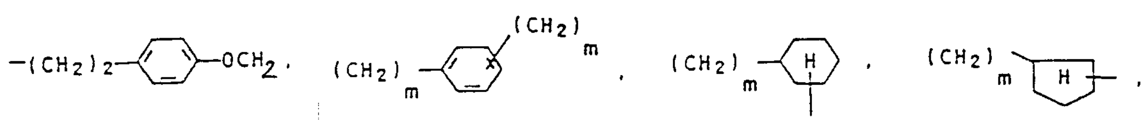

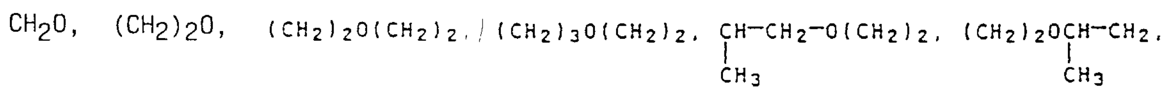

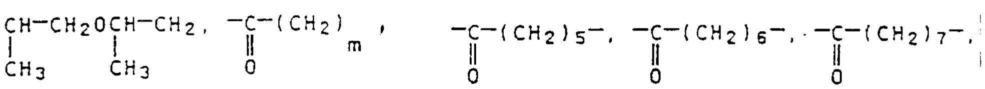

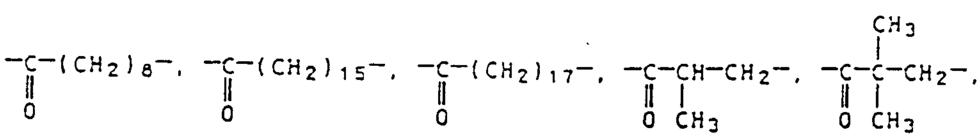

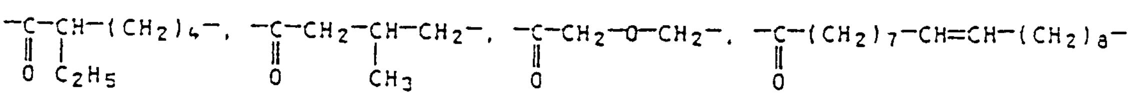

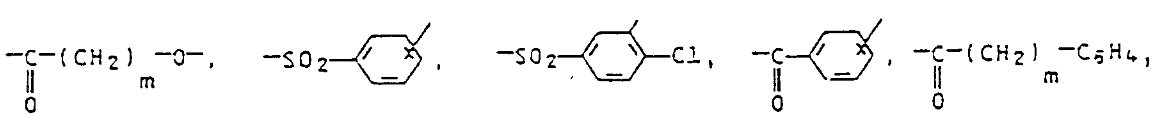

wobei p = 1 bis 20 und m 1 bis 4 ist und

Y und Z Sauerstoff, Schwefel oder $NR^8$, wobei $R^8$ Wasserstoff, $C_1$- bis $C_4$-Alkyl oder Benzyl ist, und
$R^7$ Wasserstoff
sind.

Einzelne Reste $R^1$ und $R^2$ sind neben Wasserstoff z.B.: Methyl, Ethyl, Propyl, Butyl, Pentyl, Hexyl, Benzyl, Phenylethyl, Phenylpropyl, Phenylbutyl, Methylbenzyl, Phenyl, Tolyl, Carbomethoxy, Carboethoxy, Carbopropoxy oder Carbobutoxy.

Bevorzugt sind für $R^1$ und $R^2$ Ethyl, Benzyl, Carbomethoxy oder Carboethoxy und insbesondere Wasserstoff, Methyl oder Phenyl.

Alkylreste für $R^3$ bis $R^6$ sind z.B.: $C_1$- bis $C_4$-Alkyl, also Methyl, Ethyl, Propyl oder Butyl. Zwei benachbarte Reste können dabei auch eine Tetra- oder Pentamethylengruppe bilden.

Vorzugsweise sind $R^3$ bis $R^6$ Methyl.

Bevorzugte Brückenglieder X sind z.B.:

$-(CH_2)_2-$, $-(CH_2)_3-$, $-(CH_2)_4-$, $-(CH_2)_5-$, $-(CH_2)_6-$, $-(CH_2)_7-$, $-(CH_2)_8-$, $-(CH_2)_9-$, $-(CH_2)_{10}-$, $-(CH_2)_{11}-$, $-(CH_2)_{12}-$, $-(CH_2)_{13}-$, $-(CH_2)_{14}-$, $-(CH_2)_{16}-$, $-(CH_2)_{17}-$, $-(CH_2)_{20}-$,

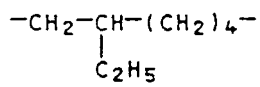, 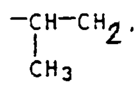

$-CH_2-CH=CH-$,

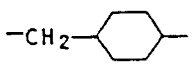, 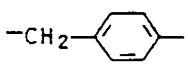, 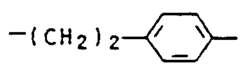, 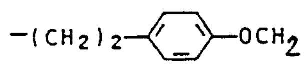, $CH_2O$,

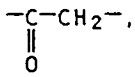, 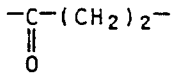, 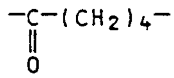, 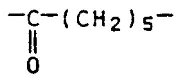, 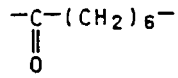, 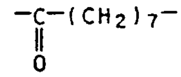,

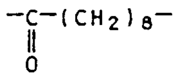, 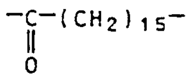, 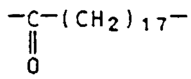, 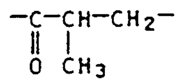, 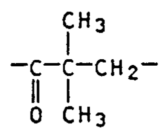,

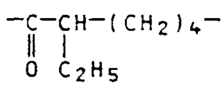, 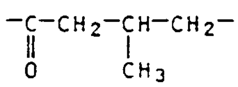, 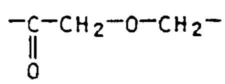, 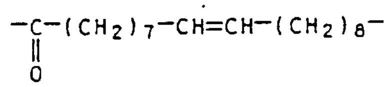,

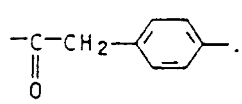

und insbesondere die direkte Bindung oder $-CH_2-$.

Reste $R^8$ sind neben Wasserstoff z.B. $C_1$- bis $C_4$-Alkyl oder Benzyl.

Verbindungen der allgemeinen Formel (I) können durch Reaktion von Tetramethylolacetylendiharnstoffen (II) analog dem Verfahren der franz. Patentschrift 22 91 203 mit 2,2,6,6-Tetraalkylpiperidin-4-aminen dargestellt werden.

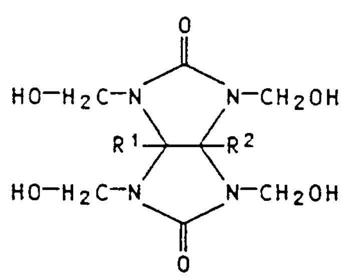 (II) ,

Verbindungen der Formel I können weiterhin im Eintopfverfahren durch Reaktion von Glykoluril, Formaldehyd und 2,2,6,6-Tetraalkylpiperidin-4-aminen analog J. Org. Chem. 50, 60 (1985) hergestellt werden.

Die Verbindungen der Formel (I) mit X-$R^7$ = H können nach literaturbekannten Verfahren z. B. durch reduktive Aminierung in die Verbindungen mit X-$R^7$ = $CH_3$ umgewandelt werden.

Verbindungen der Formel (III)

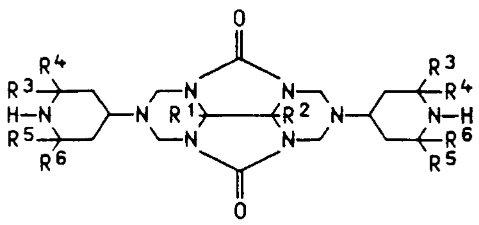

(III) ,

können weiterhin nach literaturbekannten Verfahren durch Alkylierung oder Acylierung in Verbindungen der Formel (I) mit X-$R^7$ ungleich H umgewandelt werden.

Die erfindungsgemäßen Verbindungen können in Form der freien Basen oder als Salze vorliegen. Geeignete Anionen stammen z. B. von anorganischen Säuren und insbesondere organischen Carbonsäuren sowie organischen Sulfonsäuren.

Die erfindungsgemäßen Verbindungen besitzen außerordentlich gute stabilisierende Eigenschaften, haben keine Eigenfarbe, sind gut verträglich mit organischen Polymeren und zeigen einen niedrigen Dampfdruck.

Anorganische Anionen sind z. B. Chlorid, Bromid, Sulfat, Methosulfat, Tetrafluoroborat, Phosphat oder Rhodanid.

Carbonsäure-Anionen sind beispielsweise: Formiat, Acetat, Propionat, Hexanonat, Cyclohexanonat, Lactat, Stearat, Dodecylbenzoat, Benzoat, Acrylat, Methacrylat, Zitrat, Malonat oder Succinat sowie Anionen von Polycarbonsäuren mit bis zu 3000 COOH-Gruppen.

Sulfonsäure-Anionen sind beispielsweise Benzolsulfonat oder Tosylat.

Die erfindungsgemäßen Verbindungen eignen sich zum Stabilisieren von Kunststoffen gegen den Abbau durch Licht und Wärme. Sie werden den zu stabilisierenden Kunststoffen in einer Konzentration von 0,01 bis 5 Gew.-%, vorzugsweise 0,02 bis 1 Gew.-%, vor, während oder nach der Polymerbildung zugesetzt.

Zur Vermischung der erfindungsgemäßen Verbindungen mit den zu stabilisierenden Kunststoffen können alle bekannten Vorrichtungen und Methoden zum Einmischen von Stabilisierungsmitteln oder anderen Zusätzen in Polymere angewandt werden.

Die durch eine der erfindungsgemäßen Verbindungen stabilisierten Kunststoffe können gegebenenfalls noch weitere Additive enthalten, beispielsweise Antioxidantien, Lichtstabilisierungsmittel, Metalldesaktivatoren, antistatische Mittel, flammhemmende Mittel, Pigmente und Füllstoffe.

Antioxidantien und Lichtstabilisatoren, die den Kunststoffen neben den erfindungsgemäßen Verbindungen zugesetzt werden können, sind z. B. Verbindungen auf der Basis sterisch gehinderter Phenole oder Schwefel oder Phosphor enthaltende Costabilisatoren.

Als derartige phenolische Antioxidationsmittel seien beispielsweise 2,6-Di-tert.-butyl-4-methylphenol, n-Octadecyl-β-(3,5-di-tert.-butyl-4-hydroxyphenyl)-propionat, 1,1,3-Tris-(2-methyl-4-hydroxy-5-tert.-butyl-phenyl)-butan, 1,3,5-Trimethyl-2,4,6-tris-(3,5-di-tert.-butyl-4-hydroxy-benzyl)-benzol, 1,3,5-Tris-(3,5-di-tert.-butyl-4-hydroxybenzyl)-isocyanurat, 1,3,5-Tris-[β-(3,5-di-tert.-butyl-4-hydroxyphenyl)-propionyloxy-ethyl]-isocyanurat, 1,3,5-Tris-(2,6-di-methyl-3-hydroxy-4-tert.-butylbenzyl)-isocyanurat, Pentaerythrit-tetrakis-[β-(3,5-di-tert.-butyl-4-hydroxy-phenyl)-propionat] etc. erwähnt.

Als phosphorhaltige Antioxidantien seien beispielsweise Tris-(nonyl-phenyl)-phosphit, Distearylpentaerythritdiphosphit, Tris-(2,4-di-tert.-butylphenyl)-phosphit,Tris-(2-tert.-butyl-4-methylphenyl)-phosphit, Bis-(2,4-di-tert.-butylphenyl)-pentaerythritdiphosphit, Tetrakis-(2,4-di-tert.-butylphenyl)-4,4'-biphenylendiphosphit etc. erwähnt.

Als Schwefel enthaltende Antioxidationsmittel seien beispielsweise Dilaurylthiodipropionat, Dimyristylthiodipropionat, Distearylthiodipropionat, Pentaerythrittetrakis-(β-laurylthiopropionat), Pentaerythrittetrakis-(β-hexylthiopropionat) etc. erwähnt.

Weitere Antioxidantien und Lichtstabilisatoren, die zusammen mit den erfindungsgemäßen Verbindungen verwendet werden können, sind z. B. 2-(2'-Hydroxyphenyl)-benztriazole, 2-Hydroxybenzophenone,

Arylester von Hydroxybenzoesäuren, α-Cyanozimtsäurederivate, Nickelverbindungen oder Oxalsäuredianilide.

Als organische Polymere, die durch die erfindungsgemäßen Verbindungen stabilisiert werden können, seien beispielsweise genannt:

Polymere von Mono-und Diolefinen, wie z. B. Polyethylen niedriger oder hoher Dichte, lineares Polyethylen niedriger Dichte, Polypropylen, Polyisobutylen, Polybuten-1, Polyisopren, Polybutadien sowie Copolymerisate von Mono- oder Diolefinen oder Mischungen der genannten Polymeren;

Copolymeriate von Mono- oder Diolefinen mit anderen Vinylmonomeren wie z. B. Ethylen-Alkylacrylat-Copolymere, Ethylen-Alkylmethacrylat-Copolymere, Ethylen-Vinylacetat-Copolymere oder Ethylen-Acrylsäure-Copolymere;

Polystyrol;

Copolymere von Styrol oder α-Methylstyrol mit Dienen oder Acrylderivaten, wie z. B. Styrol-Butadien, Styrol-Acrylnitril, Styrol-Ethylmethacrylat, Styrol-Butadien-Ethylacrylat, Styrol-Acrylnitril-Methacrylat;

ABS-, MBS- oder ähnliche Polymere;

Halogenhaltige Polymere, wie z. B. Polyvinylchlorid, Polyvinylfluorid, Polyvinylidenfluorid sowie deren Copolymere;

Polymere die sich von α,β-ungesättigten Säuren und deren Derivaten ableiten, wie Polyacrylate und Polymethacrylate, Polyacrylamide und Polyacrylnitrile;

Polymere, die sich von ungesättigten Alkoholen und Aminen bzw. deren Acrylderivaten oder Acetalen ableiten, wie Polyvinylalkohol oder Polyvinylacetat;

Polyurethane, Polyamide, Polyharnstoffe, Polyester, Polycarbonate, Polysulfone, Polyethersulfone und Polyetherketone.

Weitere organische Polymere, die mit den erfindungsgemäßen Verbindungen stabilisiert werden können, stellen Industrielackierungen dar. Unter diesen sind Einbrennlackierungen, unter diesen wiederum Fahrzeuglackierungen, vorzugsweise Zweischichtlackierungen, besonders hervorzuheben.

Auch hier können zusätzlich die bereits aufgeführten Antioxidantien und Lichtschutzmittel verwendet werden.

Die erfindungsgemäßen Verbindungen können, soweit sie fest sind, in fester oder gelöster Form, oder, wenn sie flüssig sind, in Substanz dem Lack zugesetzt werden. Ihre gute Löslichkeit in Lacksystemen ist dabei von besonderem Vorteil.

Bevorzugt ist die Verwendung der erfindungsgemäßen Verbindungen in Polyolefinen, vorzugsweise Ethylen- und Propylenpolymerisaten.

Von besonderer Bedeutung sind Verbindungen der allgemeinen Formel I a

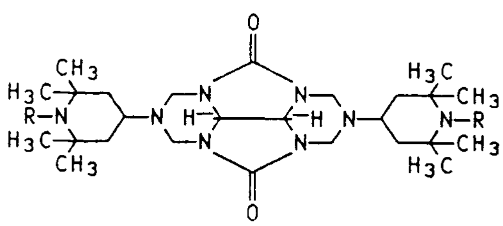

(I a) ,

in der

R Wasserstoff, Methyl, Ethyl, Propyl, Butyl, Allyl, Hydroxymethyl, Acetyl, Benzoyl oder Benzyl ist.

Beispiel 1a

Zu 100 ml n-Butanol gibt man 15,6 g (0,1 mol) 4-Amino-2,2,6,6-tetramethylpiperidin und 13,1 g (0,05 mol) Tetramethylolacetylendiharnstoff und hält die Mischung 6 h unter Rückfluß. Nach Abdestillieren des Lösungsmittels erhält man 23,2 g (92 %) farblosen Feststoff vom Schmp. 250 - 252 ° C.

| | | | |
|---|---|---|---|
| ber.: | C 62,2 | H 9,2 | N 22,3 |
| gef.: | C 62,6 | H 9,1 | N 21,7 |

Nach der Entfernung von Verunreinigungen durch Auskochen mit Wasser steigt der Schmelzpunkt auf 280 bis 283 ° C.

Beispiel 1b

Zu 300 l Wasser gibt Man 65,7 kg 4-Amino-2,2,6,6-tetramethylpiperidin und 104,8 kg einer 50 %igen wäßrigen Lösung von Tetramethylolacetylendiharnstoff. Man erhitzt 2 h auf 80 bis 90 ° C, läßt auf Raumtemperatur abkühlen, saugt ab, wäscht mit Wasser nach und trocknet im Vakuum. Man erhält 89,5 kg (90 %) farblosen Feststoff vom Schmp. 280 bis 283 ° C.

| | | | |
|---|---|---|---|
| ber.: | C 62,2 | H 9,2 | N 22,3 |
| gef.: | C 62,2 | H 9,4 | N 22,3 |

Beispiel 1c

56,8 g (= 0,4 Mol) Acetylendiharnstoff, 160 g (= 1,6 Mol) 30 %ige wäßrige Formaldehydlösung und 124,8 g (= 0,8 Mol) 4-Amino-2,2,6,6-tetramethylpiperidinwerden in 250 ml Wasser 2 h auf 90 bis 95 ° C erhitzt. Nach dem Abkühlen wird abgesaugt, mit Wasser gewaschen und getrocknet. Man erhält 186,9 g (93 %) farblosen Feststoff, der mit dem aus Beispiel 1a nach Schmp., IR und Elementaranalyse identisch ist.

Beispiel 2

Zu 17,0 g (= 0,1 Mol) 4-Amino-1,2,2,6,6-pentamethylpiperidin in 200 ml Wasser gibt man 26,2 g (=0,05 Mol) einer 50 %igen wäßrigen Lösung von Tetramethylolacetylendiharnstoff und hält 2 h auf 80 ° C. Man saugt bei Raumtemp. ab und erhält 23,8 g (90 %) farblose Kristalle vom Schmp. 257 bis 261 ° C, die zur weiteren Reinigung aus Ethanol umkristallisiert werden.

| | | | | |
|---|---|---|---|---|
| ber.: | C 63,4 | H 9,4 | O 6,0 | N 21,1 |
| gef.: | C 63,1 | H 9,4 | O 6,0 | N 21,1 |

Beispiel 3

4,95 g (= 0,019 Mol) Tetramethylolacetylendiharnstoff und 20,0 g (= 0,057 Mol) ca. 70 %iges 4-Amino-1-benzyl-2,2,6,6-tetramethylpiperidin werden in 60 ml n-Butanol 6,5 h zum Rückfluß erhitzt. Bei Raumtemperatur wird der ausgefallene Niederschlag abgesaugt, mit n-Butanol gewaschen und aus Ethanol umkristallisiert. Die Dibenzylverbindung wird als farblose Kristalle vom Schmp. 283 ° C isoliert.

| | | | | |
|---|---|---|---|---|
| ber.: | C 70,4 | H 8,5 | O 4,7 | N 16,4 |
| gef.: | C 70,1 | H 8,5 | O 4,8 | N 16,5 |

Beispiel 4

25 g (= 0,05 Mol) des Produktes aus Beispiel 1 und 40,8 g (= 0,40 Mol) Essigsäureanhydrid werden in 200 ml Xylol 5 h gekocht. Der ausgefallene Niederschlag wird bei Raumtemperatur abgesaugt, getrocknet und in Wasser gelöst. Mit Natronlauge wird die wäßrige Lösung alkalisch gemacht, der Niederschlag wird abgesaugt, getrocknet und aus Isopropanol umkristallisiert. Man erhält die Diacetylverbindung als farblosen Feststoff vom Schmp. 280 ° C.

| | | | | |
|---|---|---|---|---|
| ber.: | C 61,4 | H 8.5 | O 10,9 | N 19,1 |
| gef.: | C 61,3 | H 8,7 | O 11,1 | N 19,2 |

Beispiel 5

8,5 g (= 0,50 mmol) 1,5-Dimethyl-2,4,6,8-tetraazabicyclo[3,3,0]octan-3,7-dion, 20 ml 30 %ige wäßrige Formaldehydlösung und 15,5 g (= 100 mmol) 4-Amino-2,2,6,6-tetramethylpiperidin werden 7 h in 200 ml iso-Propanol gekocht. Das Lösungsmittel wird abdestilliert, der Rückstand mit Wasser ausgekocht. Nach dem Umkristallisieren aus Ethylenglykoldimethylether isoliert man das Produkt als farblose Kristalle vom Schmp. 279 bis 281 ° C.

| | | | | |
|---|---|---|---|---|
| ber.: | C 63,4 | H 9,4 | O 6,0 | N 21,1 |
| gef.: | C 63,2 | H 9,4 | O 6,4 | N 21,2 |

Beispiel 6

16,0 g (= 0,08 Mol) 1,5-Dimethyl-2,4,6,8-tetraazabicyclo[3,3,0]octan-3-thio-7-on, 32 ml 30 %ige Formaldehydlösung und 24,8 g (= 0,16 Mol) 4-Amino-2,2,6,6-tetramethylpiperidin werden 7 h in 200 ml iso-Propanol zum Sieden erhitzt. Das Lösungsmittel wird abdestilliert, der Rückstand mit Wasser aufgeschlämmt und abgesaugt. Nach dem Trocknen wird aus Ethylenglykoldimethylether umkristallisiert. Man isoliert das Produkt als farblosen Feststoff vom Schmp. 243 bis 245 ° C.

| | | | | | |
|---|---|---|---|---|---|
| ber.: | C 61,5 | H 9,2 | O 2,9 | N 20,5 | S 5,9 |
| gef.: | C 61,3 | H 9,4 | O 3,1 | N 20,4 | S 6,0 |

Beispiel 7

14,7 g (= 0,05 Mol) 1,5-Diphenyl-2,4,6,8-tetraazabicyclo[3,3,0]octan-3,7-dion, 20 ml 30 %ige Formaldehydlösung und 15,5 g (= 0,1 Mol) 4-Amino-2,2,6,6-tetramethylpiperidin werden 7 h in 200 ml iso-Propanol und 150 ml Dimethylsulfoxid zum Sieden erhitzt. Bei Raumtemperatur wird abgesaugt, in 250 ml Dichlormethan aufgerührt und wiederum abgesaugt. Man erhält 23,2 g (71 %) Produkt als farblosen Feststoff vom Schmp. >310 ° C.

| | | | | |
|---|---|---|---|---|
| Ber.: | C 69,7 | H 8,2 | O 4,9 | N 17,1 |
| Gef.: | C 69,5 | H 8,3 | O 5,0 | N 17,1 |

Beispiel 8

16,3 g (= 0,05 Mol) 1,5-Diphenyl-2,4,6,8-tetraazabicyclo[3,3,0]octan-3,7-dithion, 20 ml 30 %ige wäßrige Formaldehydlösung und 15,5 g (= 0,1 Mol) 4-Amino-2,2, 6,6-tetramethylpiperidin werden in 150 ml Dimethylsulfoxid 7 h auf 80 ° C und 10 h auf 150 ° C erhitzt. Bei Raumtemperatur wird abgesaugt und mit Wasser gewaschen. Das farblose Produkt hat einen Schmp. von 255 bis 256 ° C.

| | | | | |
|---|---|---|---|---|
| ber.: | C 66,5 | H 7,9 | N 16,3 | S 9,3 |
| gef.: | C 66,6 | H 7,8 | N 16,0 | S 9,1 |

Beispiel 9

Salz der Verbindung aus Beispiel 1 und 1 Mol Adipinsäure 5,0 g des Produkts aus Beispiel 1 und 1,64 g Adipinsäure werden in 150 ml Methanol gelöst. Man engt bis zur Trockne ein und erhält das mit 2 Molekülen Methanol kristallisierende Salz als farblosen Feststoff vom Schmp. 230 bis 232 ° C.

Beispiel 10

Salz der Verbindung aus Beispiel 1 mit 2 Mol 2,5-Dimethylfuran-3-carbonsäure

5,0 g des Produkts aus Beispiel 1 und 2,78 g 2,5-Dimethylfuran-3-carbonsäure werden analog Beispiel 9 umgesetzt. Man erhält das mit 2 Molekülen Methanol kristallisierende Salz als farblosen Feststoff vom Schmp. 258 bis 260 ° C.

| | | | | |
|---|---|---|---|---|
| ber.: | C 59,6 | H 8,3 | O 18,9 | N 13,2 |
| gef.: | C 59,6 | H 8,34 | N 18,0 | N 13,7 |

Beispiel 11

Salz der Verbindung aus Beispiel 1 mit 2 Mol 3-(3,5-Di-tert.-butyl-4-hydroxy-phenyl)-propionsäure.

5 g des Produkts aus Beispiel 1 und 5,7 g 3-(3,5-Di-tert.-butyl-4-hydroxy-phenyl)-propionsäure werden analog Beispiel 9 umgesetzt. Man erhält das mit 4 Molekülen-Methanol kristallisierende Salz als farblosen Feststoff vom Schmp. 208 bis 210 ° C.

| | | | | |
|---|---|---|---|---|
| ber.: | C 64,7 | H 9,4 | O 16,1 | N 9,4 |
| gef.: | C 64,4 | H 9,4 | O 15,9 | N 9,8 |

Anwendungsbeispiele

1 . Stabilisierung von Polyethylen mit der Verbindung aus Beispiel 1

a) 0,25 Teile der Verbindung aus Beispiel 1 werden in 100 Teilen Polyethylen niedriger Dichte (1840 D der Fa. BASF) durch zweimaliges Extrudieren bei 180 ° C eingearbeitet und zu 200 µm dicken Platten gepreßt. Nach 14-tägiger Lagerung im Dunkeln bei 25 ° C zeigt die Oberfläche der Platten keinen Belag.

b) Die nach a) hergestellten Platten werden in einem QUV-Schnellbewitterungs-Prüfgerät auf ihre Bewitterungsstabilität getestet. Die Alterung wird durch Messen der "CO-Zahl" nach bestimmten Zeitintervallen bestimmt. Der Beginn der Versprödung gilt als erreicht, wenn die CO-Zahl einen Wert von 10 erreicht hat. Die Testergebnisse sind in Tabelle 1 zusammengefaßt.

Analog Beispiel 1 a) wurden Prüfkörper mit den Verbindungen aus dem Beispielen 1 und 2 und dem Stabilisator der Formel IV angefertigt und analog Beispiel 1 b) getestet. Die Ergebnisse sind in Tabelle 1 zusammengefaßt.

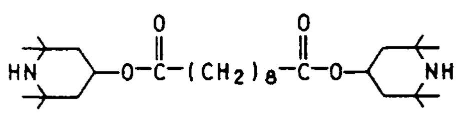

IV

Tabelle 1: CO-Zahlen bei Belichtung in einem QUV-Schnellbewitterungs-Prüfgerät (Polyethylen)

| Verbindung | Belichtungszeit in (h) 0 | 1000 | 2000 | 3000 |
|---|---|---|---|---|
| Beispiel 1 | 0,33 | 0,32 | 0,14 | 0,15 |
| Beispiel 2 | 0,08 | 0,07 | 0,11 | 0,15 |
| Stabilisator der Formel IV | nicht verträglich | | | |

2. Stabilisierung von Polypropylen

a) 0,25 Teile der Verbindung des entsprechenden Beispiels werden in 100 Teile Polypropylen (1320 H der Fa. BASF) durch zweimaliges Extrudieren bei 220 °C eingearbeitet und zu 200 µm dicken Platten gepreßt. Nach 14-tägiger Lagerung im Dunkeln bei 25 °C zeigt die Oberfläche der Platten keinen Belag.

b) Die nach a) hergestellten Platten werden in einem QUV-Schnellbewitterungs-Prüfgerät auf ihre Bewitterungsstabilität getestet. Die Al-terung wird durch Messen der "CO-Zahl" nach bestimmten Zeitintervallen bestimmt. Der Beginn der Versprödung wird mechanisch ermittelt. Die Testergebnisse sind in Tabelle 2 zusammengefaßt.

Tabelle 2: CO-Zahlen bei Belichtung in einem QUV-Schnellbewitterungs-Prüfgerät (Polypropylen)

| Verbindung | Belichtungszeit in (h) 0 | 1000 | 2000 | 3000 |
|---|---|---|---|---|
| Beispiel 1 | 3,70 | 4,34 | 46 | spröde |
| Beispiel 2 | 3,40 | 3,60 | 4,01 | 6,19 |
| Stabilisator der Formel IV | 1,65 | 1,70 | spröde | |

## Patentansprüche

**1.** Verbindungen der allgemeinen Formel I

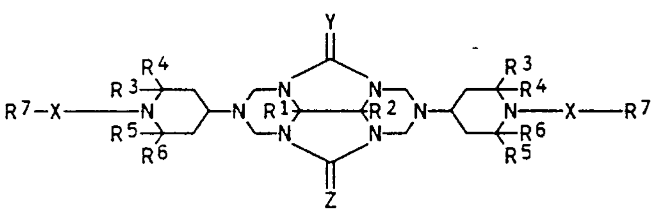

(I),

in der

$R^1$ und $R^2$ unabhängig voneinander Wasserstoff, $C_1$- bis $C_6$-Alkyl, $C_7$- bis $C_{12}$-Aralkyl, Phenyl, Tolyl, Carbomethoxy, Carboethoxy, Carbopropoxy oder Carbobutoxy,

$R^1$ und $R^2$ zusammen eine Tetra- oder Pentamethylengruppe,

$R^3$ bis $R^6$ $C_1$- bis $C_4$-Alkyl, wobei zwei benachbarte Reste dabei auch eine Tetra- oder Pentamethylengruppe bilden können, die Reste

X unabhängig voneinander eine direkte Bindung oder eine zweiwertige Gruppierung der Formel

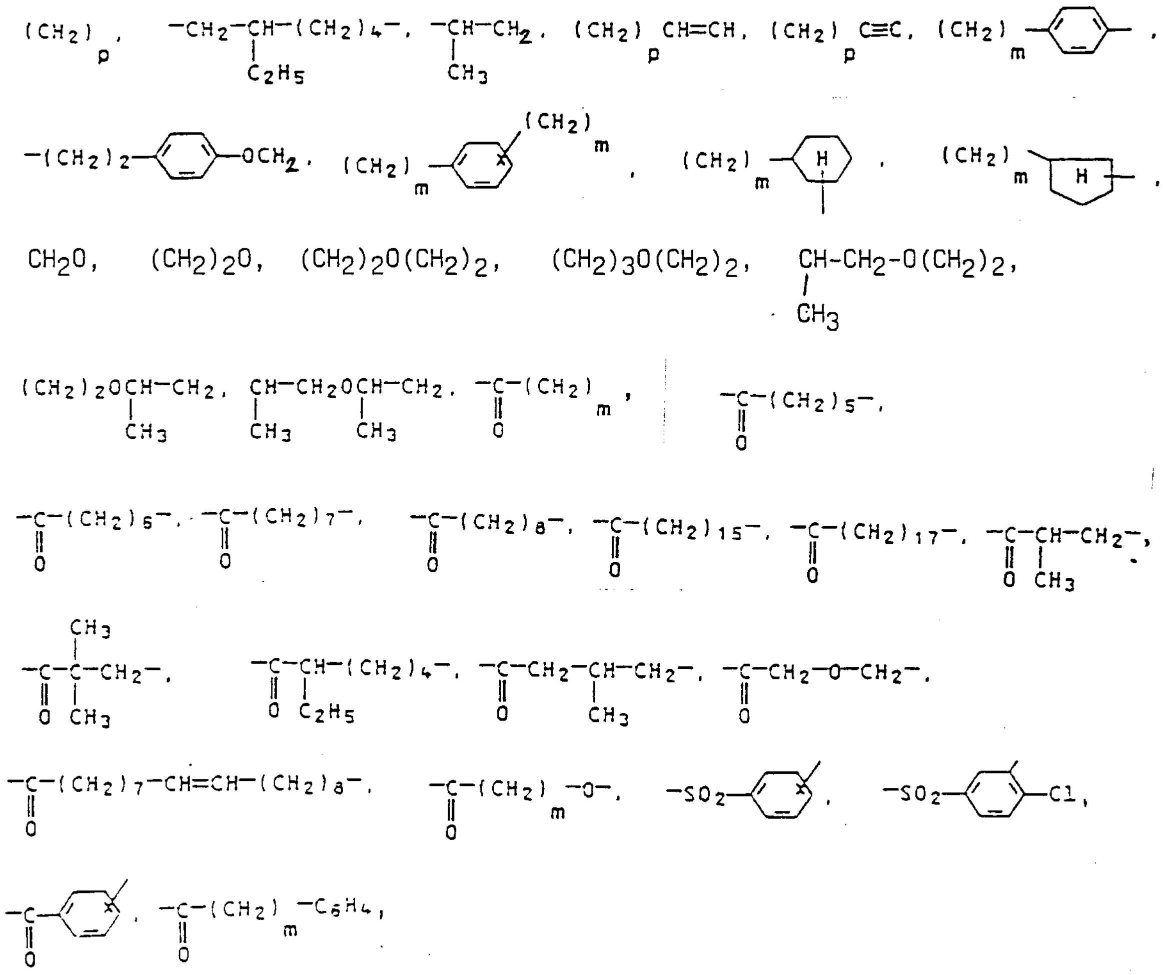

wobei p = 1 bis 20 und m 1 bis 4 ist und

Y und Z Sauerstoff, Schwefel oder $NR^8$, wobei $R^8$ Wasserstoff, $C_1$- bis $C_4$-Alkyl oder Benzyl ist, und

$R^7$ Wasserstoff

sind.

2. Verbindungen nach Anspruch 1, dadurch gekennzeichnet, daß $R^1 = R^2 = H$ ist.

3. Verbindungen nach Anspruch 1, dadurch gekennzeichnet, daß $R^1$ und/oder $R^2$ Methyl oder Phenyl sind.

4. Verbindungen nach den Ansprüchen 1 bis 3, dadurch gekennzeichnet, daß $R^3$ bis $R^6 = CH_3$ und X-$R^7$ = H sind.

5. Verbindungen nach den Ansprüchen 1 bis 3, dadurch gekennzeichnet, daß $R^3$ bis $R^6 = CH_3$ und X-$R^7$ = $CH_3$ ist.

6. Verbindungen nach Anspruch 1 der Formel Ia

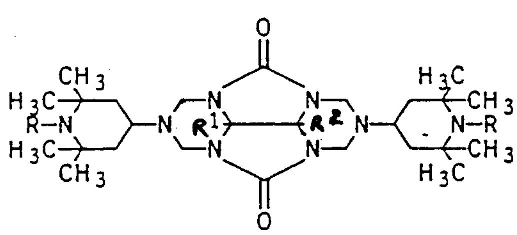

(Ia),

in der $R^1$ und $R^2$ Wasserstoff, Methyl oder Phenyl und R Wasserstoff, Methyl, Ethyl, Propyl, Butyl, Allyl, Hydroxymethyl, Acetyl, Benzoyl oder Benzyl ist.

7. Verbindungen nach Anspruch 6, bei denen R Wasserstoff oder Methyl ist.

8. Verwendung der erfindungsgemäßen Verbindungen gemäß den Ansprüchen 1 bis 7 zum Stabilisieren von Kunststoffen.

## Claims

1. A compound of the formula I

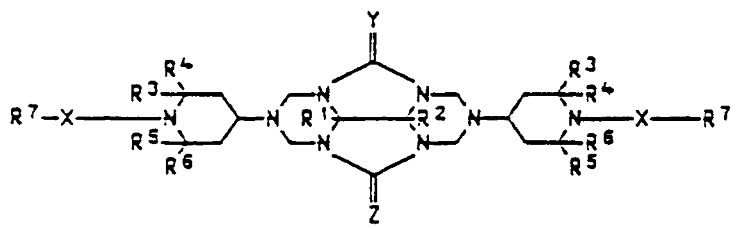

(I),

where

$R^1$ and $R^2$ independently of one another are each hydrogen, $C_1$-$C_6$-alkyl, $C_7$-$C_2$-aralkyl, phenyl, tolyl, carbomethoxy, carboethoxy, carbopropoxy or carbobutoxy, or

$R^1$ and $R^2$ together may form a tetramethylene or pentamethylene group,

$R^3$, $R^4$, $R^5$ and $R^6$ are each $C_1$-$C_4$-alkyl, where two adjacent radicals may also form a tetramethylene or pentamethylene group, the radicals independently of one another are each a direct bond or a divalent group of the formula

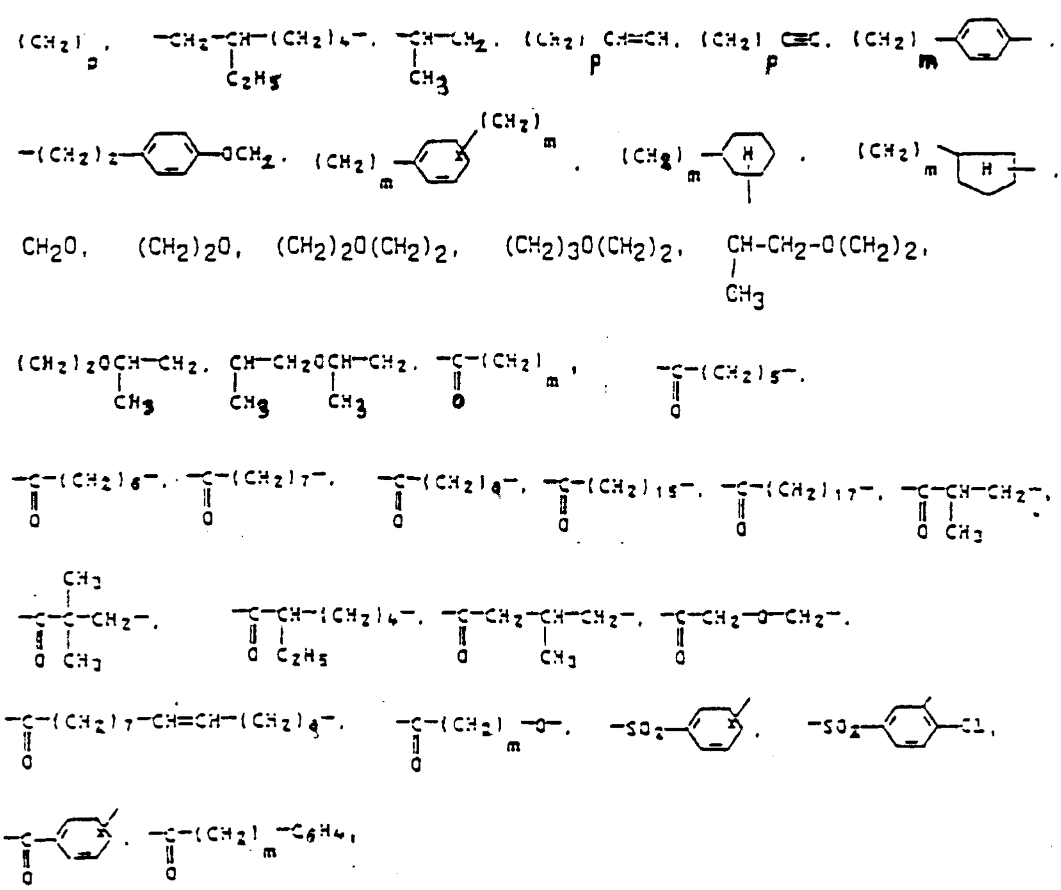

where p is 1 to 20 and m is 1 to 4, and Y and Z are each oxygen, sulfur or $NR^8$, $R^8$ being hydrogen, $C_1$-$C_4$-alkyl or benzyl, and $R^7$ is hydrogen.

**2.** A compound as claimed in claim 1, wherein $R^1$ and $R^2$ are each H.

**3.** A compound as claimed in claim 1, wherein $R^1$ or $R^2$ is methyl or phenyl, or $R^1$ and $R^2$ are each methyl or phenyl.

**4.** A compound as claimed in any of claims 1 to 3, wherein $R^3$, $R^4$, $R^5$ and $R^6$ are each $CH_3$ and X-$R^7$ is H.

**5.** A compound as claimed in any of claims 1 to 3, wherein $R^3$, $R^4$, $R^5$ and $R^6$ and X-$R^7$ are each $CH_3$.

**6.** A compound as claimed in claim 1, of the formula Ia

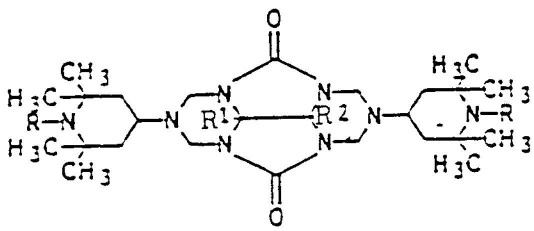

(Ia),

where $R^1$ and $R^2$ are each hydrogen, methyl or phenyl and R is hydrogen, methyl, ethyl, propyl, butyl, allyl, hydroxymethyl, acetyl, benzoyl or benzyl.

**7.** A compound as claimed in claim 6, wherein R is hydrogen or methyl.

**8.** Use of a compound according to the invention as claimed in any of claims 1 to 7 for stabilizing plastics.

**Revendications**

**1.** Composés de formule générale I

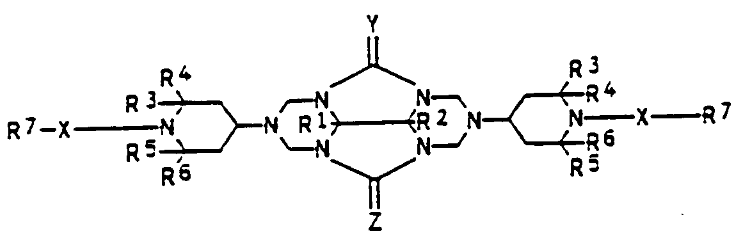

(I),

dans laquelle

- $R^1$ et $R^2$ sont chacun, independamment l'un de l'autre, un atome d'hydrogène, un reste alkyle en $C_1$ à $C_6$, aralkyle en $C_7$ à $C_{12}$, phényle, tolyle, carbométhoxy, carboéthoxy, carbopropoxy ou carbobutoxy,
- $R^1$ et $R^2$ forment ensemble un groupement tétra- ou pentamethylène,
- $R^3$ à $R^6$ dont des restes alkyle en $C_1$ à $C_4$, deux restes voisins pouvant former aussi un groupement tétra- ou pentaméthylène, les restes
- X sont chacun, independamment l'un de l'autre, une liaison directe ou un groupement bivalent de formule

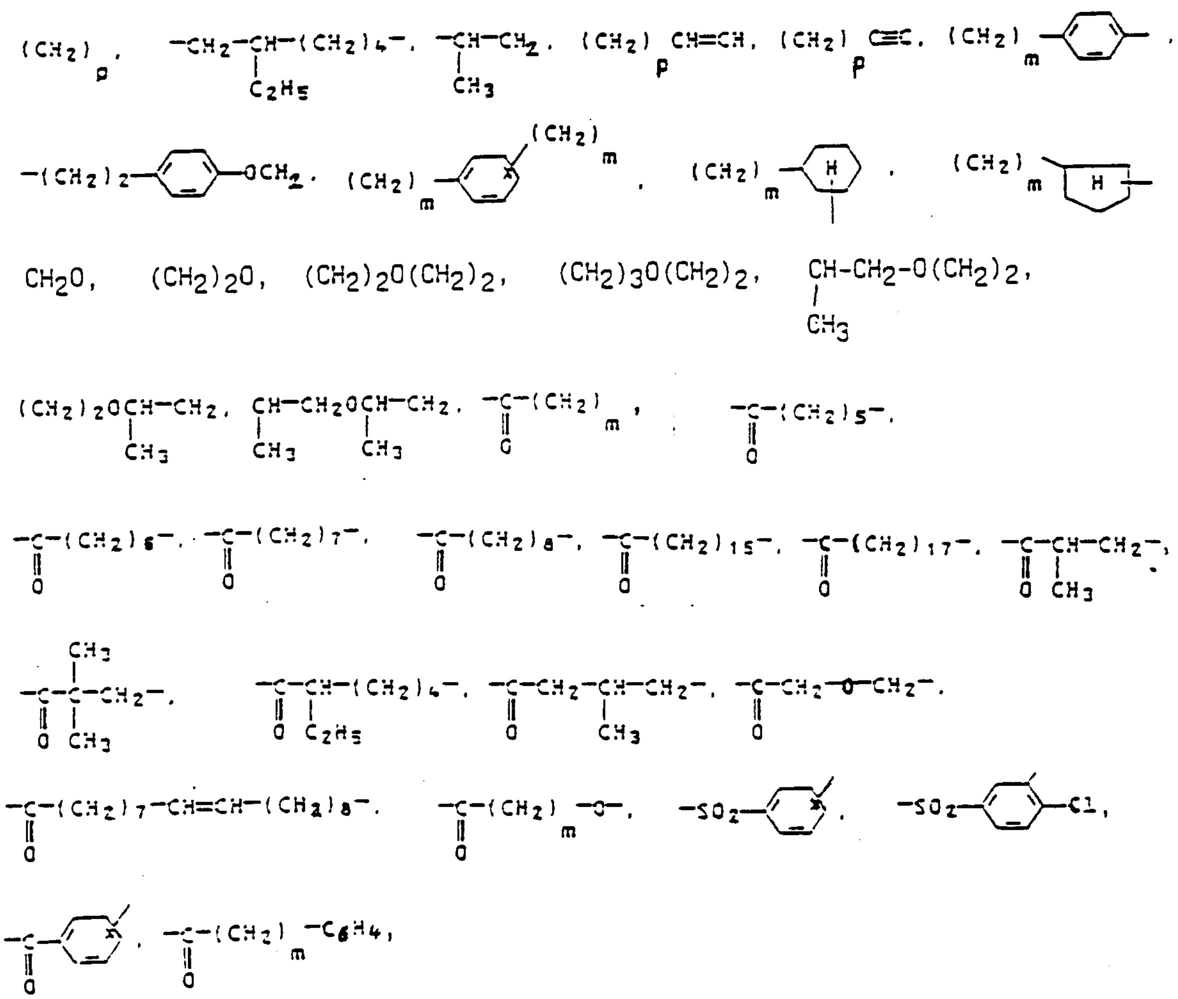

où p = 1 à 20 et m = 1 à 4,

Y et Z sont chacun un atome d'oxygène, de soufre ou un groupement $NR^8$, $R^8$ étant un atome d'hydrogène ou un reste alkyle en $C_1$ à $C_4$ ou benzyle, et

$R^7$ est un atome d'hydrogène.

**2.** Composés selon la revendication 1, caractérisés en ce que $R^1 = R^2 = H$

**3.** Composés selon la revendication 1, caractérisés en ce que $R^1$ et/ou $R^2$ sont des restes méthyle ou phényle.

**4.** Composés selon l'une quelconque des revendications 1 à 3, caractérisés en ce que $R^3$ à $R^6 = CH_3$ et $X\text{-}R^7 = H$.

**5.** Composés selon l'une quelconque des revendications 1 à 3, caractérisés en ce que $R^3$ à $R^6 = CH_3$ et $X\text{-}R^7 = CH_3$.

**6.** Composés selon la revendication 1 de formule Ia

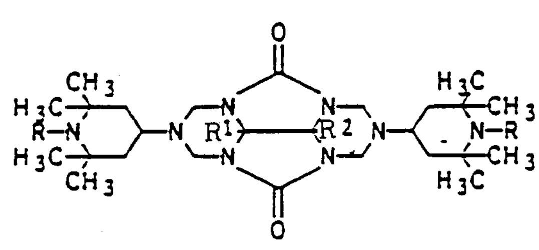

(Ia),

dans laquelle $R^1$ et $R^2$ sont chacun un atome d'hydrogène ou un reste méthyle ou phényle, et R est un atome d'hydrogène ou un reste méthyle, éthyle, propyle, butyle, allyle, hydroxyméthyle, acétyle, benzoyle ou benzyle.

**7.** Composés selon la revendication 6, dans lesquels R est un atome d'hydrogène ou un reste méthyle.

**8.** Utilisation des composés de l'invention selon l'une quelconque des revendications 1 à 7 pour la stabilisation de matières plastiques.